# EUROPEAN PATENT APPLICATION

(11) **EP 4 062 873 A1**
(43) Date of publication of application: **28.09.2022**
(21) Application number: 20890245.2
(22) Date of filing: 23.09.2020
(51) Int. Cl.: A61F 2/24

(54) **INDEPENDENTLY CONTROLLABLE VALVE CLAMPING SYSTEM**

(30) Priority: 19.11.2019 CN 201911138749; 19.11.2019 CN 201922008013 U
(71) Applicant: Hangzhou Valgen Medtech Co., Ltd., Hangzhou, Zhejiang 310052 (CN)
(72) Inventor: ZHANG, Tingchao, Hangzhou, Zhejiang 310052 (CN); WANG, Zetao, Hangzhou, Zhejiang 310052 (CN); ZHANG, Weiwei, Hangzhou, Zhejiang 310052 (CN)
(74) Representative: Hamer, Christopher K.
(86) International application number: PCT/CN2020/117249
(87) International publication number: WO 2021/098371

(57) **Abstract**

A valve clamping system capable of being independently controlled is provided. The valve clamping system includes a valve clip (100) and a delivery device (200) configured to deliver the valve clip (100). The valve clip (100) includes a fixing base (10) and proximal clamping pieces (20) capable of being folded or unfolded relative to the fixing base (10). The delivery device (200) includes a delivery sheath (210) and control parts (230, 230b, 230c). The delivery sheath (210) defines at least one pair of first cavities (211) that are symmetrical about an axis of the delivery sheath (210) and axially extend, and each control part (230, 230b, 230c) movably threads the at least one pair of first cavities (211). Each control part (230, 230b, 230c) is connected to one corresponding proximal clamping piece (20), so as to control the proximal clamping pieces (20) to be folded or unfolded relative to the fixing base (10). When the control part (230, 230b, 230c) is tensioned toward a proximal end to control the corresponding proximal clamping piece (20) to be folded relative to the fixing base (10), a resultant of pulling forces, applied by each control part (230, 230b, 230c), on the corresponding proximal clamping pieces (20) is on an axis of the delivery sheath (210), thereby avoiding bending of the delivery sheath (210) and swinging of the valve clip (100), and facilitating reduction of operation time and improvement of operation efficiency, and avoiding damage to patients.

## Description

### TECHNICAL FIELD

This application relates to the field of medical instruments, and in particular to a valve clamping system capable of being independently controlled.

### BACKGROUND

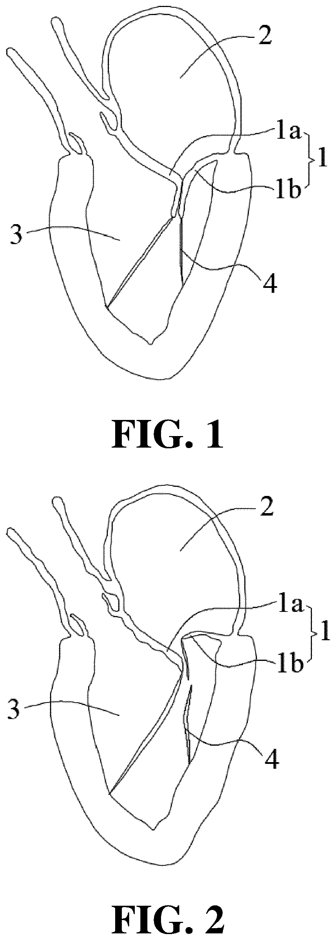

Referring to FIG. 1, a mitral valve 1 is a one-way valve between a left atrium 2 and a left ventricle 3 of the heart. The normal and healthy mitral valve 1 can control blood to flow from the left atrium 2 to the left ventricle 3, while preventing the blood from flowing from the left ventricle 3 to the left atrium 2. The mitral valve 1 has a pair of leaflets, called an anterior leaflet 1a and a posterior leaflet 1b. The anterior leaflet 1a and the posterior leaflet 1b are fixed on a papillary muscle of the left ventricle 3 through a chordae tendineae 4. Under normal circumstances, during systole of the left ventricle 3 of the heart, edges of the anterior leaflet 1a and the posterior leaflet 1b are in full coaptation to prevent the blood from flowing from the left ventricle 3 to the left atrium 2. Referring to FIG. 2, when the mitral valve leaflets or associated structures thereof have organic or functional lesion (for example, partial rupture of the chordae tendineae 4), insufficient coaptation of the anterior leaflet 1a and the posterior leaflet 1b of the mitral valve 1 may be caused. During systole of the left ventricle 3 of the heart, the mitral valve 1 cannot be closed sufficiently, so that the blood flows back from the left ventricle 3 to the left atrium 2, thereby causing a series of pathological and physiological changes, which are referred to as "mitral valve regurgitation".

There is a minimally invasive surgery, which is based on the principle of edge to edge surgery of valves. A valve clamp is delivered to the mitral valve through an intervention catheter, and then the anterior and posterior leaflets of the mitral valve are simultaneously clamped by relatively opening and closing the clamp, so that the anterior and posterior leaflets are pulled to each other, achieving alleviation of "mitral valve regurgitation". During clamping of the leaflets, two leaflets of the mitral valve are kept in a state of large-range and intensive opening and closing movement, resulting in a great difficulty in clamping. Therefore, it is usually desirable to control the clamping action of the leaflet on each side individually.

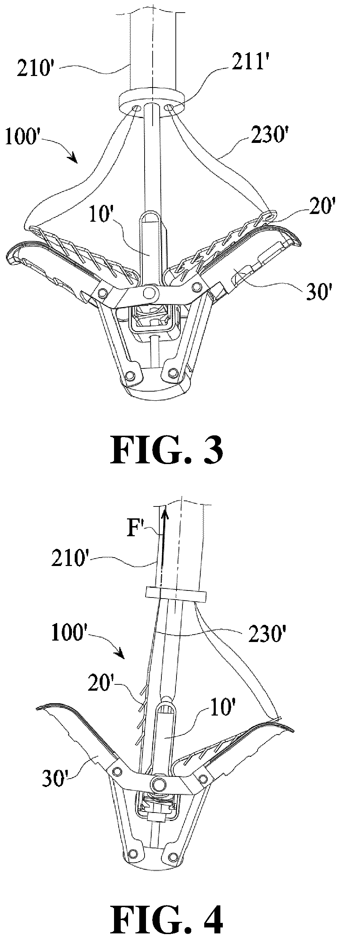

Referring to FIG. 3, a valve clamp 100' is provided and includes two pairs of proximal clamping pieces 20' and distal clamping pieces 30' that are respectively located on two opposite sides of an axis of a delivery sheath 210'. Each proximal clamping piece 20' is controlled to move by a control wire 230' threading the same. The two proximal clamping pieces 20' can be individually released and expanded relative to a fixing base 10' under action of their resilience to move closer to the corresponding distal clamping pieces 30', so as to respectively clamp the two leaflets of the mitral valve. However, as shown in FIG. 4, in the related art, incoming and outgoing of the control wire 230' of each proximal clamping piece 20' are achieved through a same cavity 211' disposed on one side of the axis of the delivery sheath 210'. During repeated operative procedures, when the proximal clamping piece 20' on one single side is tensioned by the control wire 230' to be closed relative to the fixing base 10', a pulling force F' applied on the proximal clamping piece 20' is exerted on one side of the axis of the delivery sheath 210'. The pulling force F' may generate a certain torque to the valve clamp 100', causing the valve clamp 100' to swing to the side applied with the pulling force F', and further resulting in bending of the delivery sheath 210'. Therefore, in order to accurately release the proximal clamping pieces 20' to re-clamp the leaflets of the mitral valve, the position of the valve clamp 100' may need to be adjusted frequently by an operator, which leads to a relatively long operation time and a relatively low efficiency. In addition, the valve clamp 100' that swings may pull the leaflets or hook the chordae tendineae, and then tear the leaflets or break the chordae tendineae, which will not only cause failure of operations, but even cause serious injury to patients.

### SUMMARY

In view of this, a valve clamping system capable of being independently controlled is provided in this application, which can not only individually clamp the leaflet on one side but also avoid bending of a delivery sheath and swinging of a valve clip, thereby facilitating reduction of operation time and improvement of operation efficiency, and avoiding damage to patients.

In order to solve the above problem, a valve clamping system capable of being independently controlled is provided. The valve clamping system includes a valve clip and a delivery device configured to deliver the valve clip. The valve clip includes a fixing base and proximal clamping pieces capable of being folded or unfolded relative to the fixing base. The delivery device includes a delivery sheath and control parts. Each control part is connected to one corresponding proximal clamping piece, so as to control the proximal clamping pieces to be folded or unfolded relative to the fixing base. The delivery sheath defines at least one pair of first cavities that are symmetrical about an axis of the delivery sheath and axially extend, and each control part movably threads the at least one pair of first cavities.

In the valve clamping system capable of being independently controlled, the control part threads the at least one pair of first cavities of the delivery sheath which are disposed symmetrically about the axis of the delivery sheath. When the control parts control the corresponding proximal clamping pieces to be folded relative to the fixing base, the resultant of the pulling forces, applied by the control parts, on the corresponding proximal clamping pieces is exerted on the axis of the delivery sheath, thereby avoiding bending of the delivery sheath and swinging of the valve clip, and facilitating reduction of operation time and improvement of operation efficiency, and avoiding damage to patients.

### BRIEF DESCRIPTION OF THE DRAWINGS

In order to describe the technical solutions in embodiments of this application more clearly, the accompanying drawings required to be used in the embodiments will be simply introduced below. Apparently, the accompanying drawings in the following descriptions are some embodiments of this application. Those of ordinary skill in the art may further obtain other accompanying drawings according to these accompanying drawings without creative work.
FIG. 1 is a schematic diagram of a mitral valve in a normal state.
FIG. 2 is a schematic diagram of a mitral valve with a lesion.
FIG. 3 is a schematic perspective view of a valve clip and a delivery sheath in the related art.
FIG. 4 is a schematic view showing a tensioned state of a proximal clamping piece on a single side of the valve clip in FIG. 3.
FIG. 5 is a schematic perspective view of a valve clamping system provided in a first embodiment of this application.
FIG. 6 is a schematic view showing a tensioned state of a proximal clamping piece on a single side of the valve clip in FIG. 5.
FIG. 7 is a schematic perspective view of a partial structure of the valve clamping system in FIG. 5.
FIG. 8 is a schematic perspective view of a fixing base in FIG. 7.
FIG. 9 is a schematic perspective view of proximal clamping pieces in FIG. 7.
FIG. 10 is a schematic view showing an application state of the valve clip in FIG. 5.
FIG. 11 is a schematic view of the mitral valve during systole after the leaflets are clamped by the valve clip in FIG. 10.
FIG. 12 is a schematic view of the mitral valve during diastole after the leaflets are clamped by the valve clip in FIG. 10.
FIG. 13 is a schematic view of a distal end face of the delivery sheath in FIG. 5.
FIG. 14 and FIG. 15 are schematic views of distal end faces of delivery sheaths in other embodiments.
FIG. 16 is a schematic perspective view of a part of a delivery device in FIG. 5.
FIG. 17 is a sectional view of the delivery device in FIG. 16.
FIG. 18 to FIG. 21 are schematic views showing use of the valve clamping system in FIG. 5.
FIG. 22 is a schematic perspective view of a partial structure of a valve clamping system provided in a second embodiment of this application.
FIG. 23 is a schematic perspective view of a partial structure of a valve clamping system provided in a third embodiment of this application.
FIG. 24 is a schematic structural view of a valve clamping system provided in a fourth embodiment of this application.

### DETAILED DESCRIPTION

The technical solutions in the implementations of the present application are clearly and completely described in the following with reference to the accompanying drawings in the implementations of the present application. Apparently, the described implementations are merely a part of rather than all the implementations of the present application. All other implementations obtained by those of ordinary skill in the art based on the implementations of the present application without creative efforts are within the scope of the present application.

It is noted that terms such as "on", "under", "in", "out", and the like referred to herein which indicate directional relationship or positional relationship are directional relationship or positional relationship based on accompanying drawings and are only for the convenience of description and simplicity, rather than explicitly or implicitly indicate that apparatuses or components referred to herein must have a certain direction or be configured or operated in a certain direction and therefore cannot be understood as limitation on the disclosure. In addition, terms "first", "second", "third", and the like are only used for description and cannot be understood as explicitly or implicitly indicating relative importance or implicitly indicating.

In the description of this application, it is to be noted that in the field of interventional medical instruments, "proximal end" indicates one end close to an operator, "distal end" indicates one end farther away from the operator, and "axial direction" indicates a direction parallel to a line between a distal center and a proximal center of a medical instrument in a natural state. The above definitions are only for the convenience of presentation and should not be understood as limits to this application.

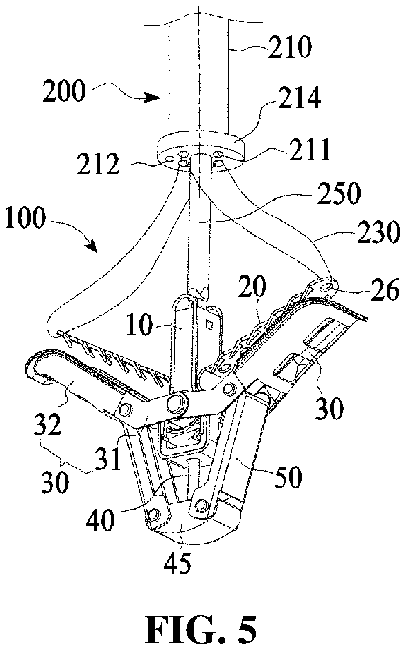

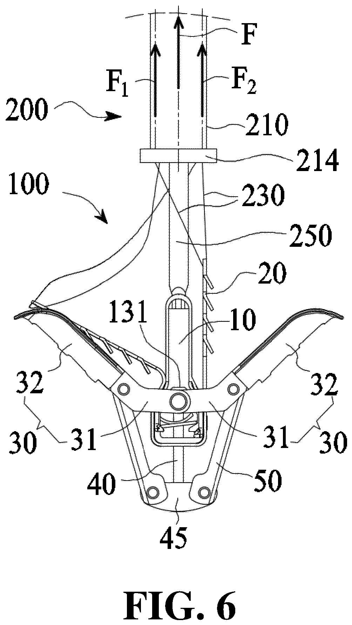

Referring to FIG. 5 and FIG. 6 together, a valve clamping system capable of being independently controlled is provided in a first embodiment of this application and includes a valve clip 100 and a delivery device 200 configured to deliver the valve clip 100. The valve clip 100 includes a fixing base 10 and proximal clamping pieces 20 capable of being folded or unfolded relative to the fixing base 10. The delivery device 200 includes a delivery sheath 210 and control parts 230. Each control part 230 is connected to one corresponding proximal clamping piece 20, so as to control the proximal clamping pieces 20 to be folded or unfolded relative to the fixing base 10. Specifically, the delivery sheath 210 defines at least one pair of first cavities 211 that are symmetrical about an axis of the delivery sheath 210 and axially extend. Each control part 230 movably threads the at least one pair of first cavities 211. Thus, when the control part 230 is tensioned toward a proximal end to control the corresponding proximal clamping piece 20 to be folded relative to the fixing base 10, a resultant F of pulling forces, applied by each control part 230, on the corresponding proximal clamping piece 20 is on the axis of the delivery sheath 210, thereby avoiding bending of the delivery sheath 210 and swinging of the valve clip 100.

The valve clip 100 further includes a driving component that is rotationally connected to distal clamping pieces 30 of the fixing base 10 and used to drive the distal clamping pieces 30 to be unfolded or folded relative to the fixing base 10. The driving component includes a push rod 40 that is axially and slidably disposed in the fixing base 10, and link rods 50 each connected to the distal clamping pieces 30 and the push rod 40. When the push rod 40 axially slides relative to the fixing base 10, the distal clamping pieces 30 are unfolded relative to the fixing base 10 under pulling of the link rods 50. After the distal clamping pieces 30 are disposed at proper positions below the valve, the proximal clamping pieces 20 are released from control of the control parts 230. The proximal clamping pieces 20 are released to be expanded relative to the fixing base 10, and then move closer to the distal clamping pieces 30 to clamp the valve between the proximal clamping pieces 20 and the distal clamping pieces 30. When a problem of unstable clamping of the valve occurs, the control parts 230 can be tensioned by the operator to control the corresponding proximal clamping pieces 20 to be closed relative to the fixing base 10, so that the proximal clamping pieces 20 can be released again to re-clamp the valve.

In this application, each control part 230 threads the at least one pair of first cavities 211 of the delivery sheath 210 which are disposed symmetrically about the axis of the delivery sheath 210. When the control part 230 controls the corresponding proximal clamping piece 20 to be closed relative to the fixing base 10, the resultant F of the pulling forces, applied by the control part 23, on the corresponding proximal clamping piece 20 is exerted on the axis of the delivery sheath 210. As such, the resultant F generates a torque of zero to the valve clip 100. Thus, bending of the delivery sheath 210 and swinging of the valve clip 100 can be avoided, and the proximal clamping pieces 20 can be quickly and accurately released by the operator to clamp the valve, without frequently adjusting the position of the valve clip 100, which facilitates reduction of operation time and improvement of operation efficiency, and prevents the valve clip 100 from swinging to hurt a patient.

In this embodiment, both the number of the proximal clamping pieces 20 and the distal clamping pieces 30 are two. The two proximal clamping pieces 20 and the two distal proximal clamping pieces 30 are in one to one correspondence to form two clamps. The two clamps are in axial symmetry about the fixing base 10. The valve clip 100 is delivered to a mitral valve of the patient through the delivery device 200. The two clamps can respectively clamp the anterior and posterior leaflets of the mitral valve to reduce a gap between the leaflets, and remain in the body of the patient as an implant, thereby achieving alleviation or treatment of "mitral valve regurgitation".

In order to ensure safety after implantation, the fixing base 10, the proximal clamping pieces 20, and the distal clamping pieces 30 are respectively made of a biocompatible metal material such as stainless steel, cobalt alloy, cobalt-chromium alloy, titanium alloy, or nickel-titanium alloy. The push rod 40 and the link rods 50 are respectively made of a biocompatible polymer material or a metal material such as polyester, silicone resin, stainless steel, cobalt alloy, cobalt-chromium alloy, or titanium alloy. Preferably, in order to improve a clamping force, in this embodiment, the fixing base 10, the distal clamping pieces 30, the push rod 40, and the link rods 50 are all made of stainless steel or cobalt-chromium alloy with a higher hardness, and the proximal clamping pieces 20 are made of a material having a shape memory function, preferably super-elastic nickel-titanium alloy.

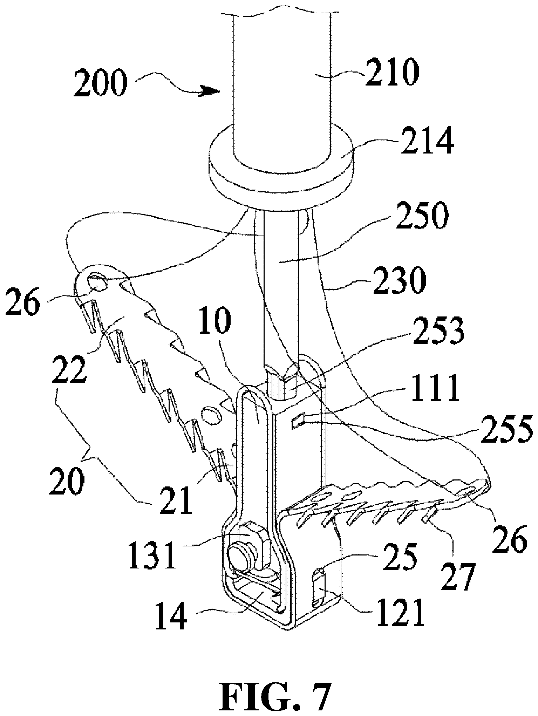

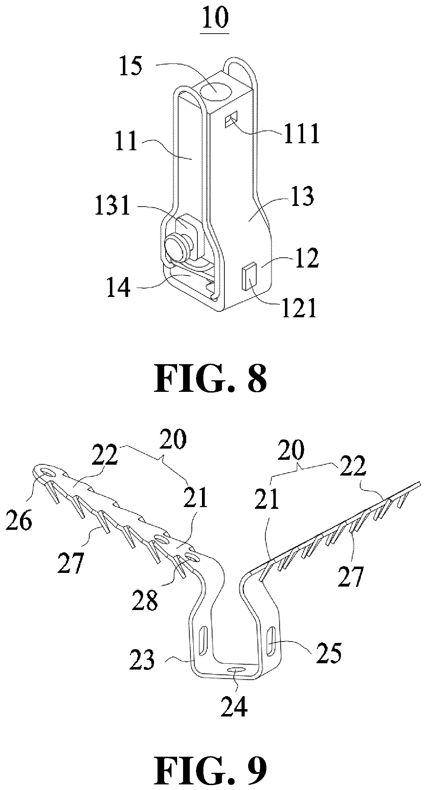

Specifically, referring to FIG. 7 and FIG. 8 together, the fixing base 10 includes a first base body 11 at a proximal end of the fixing base 10, a second base body 12 at a distal end of the fixing base 10, and a third base body 13 used for transitional connection between the first base body 11 and the second base body 12. The first base body 11, the second base body 12, and the third base body 13 may be of an integrated structure or a non-integrated structure. In this embodiment, the three are the integrated structure.

The first base body 11 defines a tubular cavity which axially extends through two end faces. Two rectangular locking holes 111 that communicate with the tubular cavity and are symmetrical about an axis of the first base body 11 are defined at the proximal end of the first base body 11 and used to be connected with a connecting rod 250 of the delivery device 200.

The second base body 12 defines an accommodating cavity 14 in a direction perpendicular to an axial direction of the second base body 12, and the accommodating cavity 14 extends through two opposite side surfaces of the second base body 12. The second base body 12 defines a through hole, which extends axially, in an inner wall of a distal end of the accommodating cavity 14. An axis of the through hole and an axis of the tubular cavity of the first base body 11 are collinear. Other two opposite side surfaces of the second base body 12 are respectively and protrusively provided with a rectangular block 121.

Two opposite planes of the third base body 13 are respectively and protrusively provided with a joint block 131. The joint block 131 defines a pin hole that is used for mounting a pivot pin or a bolt.

Furthermore, the third base body 13 defines a through hole (not shown in the figure) in an axial direction. The tubular cavity of the first base body 11, the through hole of the third base body 13, the accommodating cavity 14 of the second base body 12, and the through hole defined at the inner wall of the distal end of the accommodating cavity 14 are coaxially communicated to form a threading passage 15.

Referring to FIG. 5 and FIG. 6 together, the driving component includes the push rod 40. The push rod 40 slidably threads the threading passage 15 of the fixing base 10 in the axial direction. The proximal end of the push rod 40 is connected to a mandrel (not shown in the figure) of the delivery device 200. The distal end of the push rod 40 is provided with a connecting base 45. Two opposite ends of the connecting base 45 define two pin holes that extends through two opposite ends respectively. The connecting base 45 is of any structure in a shape such as a hemisphere, a spherical crown, or a bullet shape, so that the valve clip 100 can be delivered more easily in the body.

It is to be noted that a locking part is disposed in the accommodating cavity of the fixing base 10, and adjusting parts are respectively disposed on two opposite side surfaces of the fixing base 10. With matching between the locking part and the adjusting parts, the push rod 40 can be fixed or unlocked relative to the fixing base 10. Specifically, the locking part may be a combination of a deformable elastic piece and a steel sheet in the related art, and the adjusting parts may be metal wires made of nickel-titanium alloy or the like. Since they are unrelated to modification and creation of this application, elaboration is omitted here.

Furthermore, as shown in FIG. 5 and FIG. 6, in this embodiment, the driving component further includes two link rods 50 disposed opposite to each other. Each link rod 50 is used to connect the distal clamping piece 30 on the corresponding side to the connecting base 45 at the distal end of the push rod 40. When the push rod 40 axially slides in the fixing base 10, the distal clamping piece 30 can be pulled to be opened or closed relative to the fixing base 10 by the link rod 50.

Specifically, each distal clamping piece 30 includes a connecting section 31 at a distal end of the distal clamping piece 30 and a clamping section 32 connected to a proximal end of the connecting section 31. An end of the connecting section 31 away from the corresponding clamping section 32 is rotationally connected to the joint block 131 of the third base body 13 of the fixing base 10. An end of the connecting section 31 close to the clamping section 32 is rotationally connected to a proximal end of the link rod 50 on the corresponding side. A distal end of this link rod 50 is rotationally connected to the pin hole of the connecting base 45 through a pivot pin or a bolt.

In this application, with the matching between the locking part and the adjusting parts of the fixing base 10, the push rod 40 is unlocked relative to the fixing base 10, and the push rod 40 may slide to the distal end in the axial direction to move relative to the fixing base 10. Therefore, the connecting base 45 at the distal end of the push rod 40 moves relative to the fixing base 10, the connecting base 45 drives the link rods 50 to move, and under pulling of the link rods 50, the distal clamping pieces 30 may rotate around the centers of the pin holes on the joint blocks 131 to be opened relative to the fixing base 10. When the proximal clamping pieces 20 are released, a leaflet accommodating space is defined between each proximal clamping piece 20 and the corresponding distal clamping piece 30, and the proximal clamping piece 20 moves closer to the corresponding distal clamping piece 30 to clamp the valve between the two. After the proximal clamping piece 20 and the distal clamping piece 30 clamp the valve, the push rod 40 is driven to axially slide to the proximal end, and further the link rods 50 drive the distal clamping pieces 30 to be closed relative to the fixing base 10, until the distal clamping pieces 30 are fully closed relative to the fixing base 10. At this point, the valve clip 100 is in a closing state. With the matching between the locking part and the adjusting parts again, the push rod 40 is fixed relative to the fixing base 10 to prevent the distal clamping pieces 30 to be opened relative to the fixing base 10, and the valve clip 100 in the closing state is hung under the valve.

When the link rods 50 drive the distal clamping pieces 30 to be opened or closed relative to the fixing base 10, the distal clamping pieces 30 may be opened or closed within a larger range relative to the fixing base 10, and an included angle between the two distal clamping pieces 30 may reach a maximum of 300 degrees. That is, after the distal clamping pieces 30 are opened relative to the fixing base 10, the distal clamping pieces 30 can be turned down to some extent, thereby facilitating clamping of the valve that constantly moves and increasing the success rate of clamping. In this embodiment, the included angle between the two distal clamping pieces 30 is preferably in a range of 0 degree to 240 degrees, more preferably 120 degrees to 180 degrees.

Preferably, a first surface of the distal clamping piece 30 facing the proximal clamping piece 20 may be provided with a clamping anti-slip structure (not shown) to enhance a friction force when the distal clamping piece 30 is in contact with the valve, thereby providing a stable clamping force while preventing the distal clamping piece 30 from damaging the valve. The clamping anti-slip structure may be protrusions or slots that are disposed on the first surface, or may be a pad that fits the first surface and made of a biocompatible material with a higher friction coefficient.

The first surface may be a flat surface or a curved screen. Preferably, the first surface is a curved surface for increasing a contact area and a clamping area between the distal clamping piece 30 and the valve, thereby providing a stable clamping force. In addition, an accommodating slot is formed on the curved first surface, when the proximal clamping piece 20 moves closer to the distal clamping piece 30, at least part of the proximal clamping piece 20 is accommodated in the accommodating slot, so that the valve between the proximal clamping piece 20 and the distal clamping piece 30 is clamped, and the size of the valve clip 100 in a closing state is reduced to the utmost extent, which is conductive to delivery of the valve clip 100 in the body.

Further preferably, the first surface of the distal clamping piece 30 may be applied with an active drug or define at least one opening, to promote crawling and growth of endothelial cells that are located on an inner surface of the distal clamping piece 30 and of the valve tissue. Moreover, forming of the opening may reduce the overall weight of the valve clip 100, thereby preventing the valve clip 100 that is heavy excessively and hung below the leaflets for a long time from slipping off or causing damage to the leaflets.

Referring to FIG. 5, FIG. 7, and FIG. 9 together, each proximal clamping piece 20 includes a connecting end 21 and a free end 22 arranged opposite to the connecting end 21, and the connecting end 21 is fixed relative to the fixing base 10. In this embodiment, the two proximal clamping pieces 20 are connected integrally through a connecting frame 23. The connecting frame 23 defines a through hole 24 for the push rod 40 to extend through. The connecting frame 23 defines rectangular holes 25 at two opposite sides for the rectangular blocks 121 on the second base body 12 to extend through. The connecting frame 23 is sleeved on the second base body 12 and the third base body 13 to fix the connecting ends 21 of the two proximal clamping pieces 20 to the fixing base 10. In other embodiments, the connecting ends 21 of the proximal clamping pieces 20 may be fixed to the fixing base 10 by welding, crimping, or the like.

At least part of each proximal clamping piece 20 is made of a material having a shape memory function. After heat setting treatment, in a natural state, the proximal clamping piece 20 extends radially outwards relative to the fixing base 10, and preferably extends to the distal end, thereby being matched with the distal clamping piece 30 to clamp the leaflet. In this embodiment, the proximal clamping piece 20 is prepared by placing cut nickel-titanium alloy in a setting mold, performing heat treatment on the setting mold under a condition of 300-650 °C, taking out to cool rapidly, and removing the setting mold, such that the shaped proximal clamping piece 20 is obtained. Specifically, in this embodiment, the proximal clamping piece 20 is made of super-elastic nickel-titanium alloy, and the connecting frame 23 is also made of nickel-titanium alloy and is integrally formed with the proximal clamping piece 20, so that the difficulty of a production process is reduced, a process flow is simplified, and production cost is lowered. In other embodiments, the connecting frame 23 may also be made of stainless steel and be welded to the proximal clamping pieces 20, so that connection strength of the connecting frame 23 and the second base body 12 is improved.

Each proximal clamping piece 20 is provided with at least one connecting structure 26 that is used to be connected to a corresponding control part 230. Specifically, as shown in FIG. 7, in this embodiment, each proximal clamping piece 20 defines a connecting hole. The connecting hole is the connecting structure 26. The control part 230 threads the connecting hole of the corresponding proximal clamping piece 20, and extends out of the body of the patient through the delivery sheath 210. The corresponding proximal clamping piece 20 is controlled through the control part 230 of an external part by the operator to be closed or opened relative to the fixing base 10. Preferably, in this embodiment, the connecting structure 26 is disposed at an end (i.e., the free end 22), where the end of the proximal clamping piece 20 is away from the fixing base 10 when the proximal clamping piece 20 is opened relative to the fixing base 10, thereby helping the operator to control the proximal clamping piece 20 to be closed relative to the fixing base 10 with a smaller pulling force.

In other embodiments, the connecting structure 26 may be two or at least two connecting holes defined in each proximal clamping piece 20, and each control part 230 sequentially threads multiple connecting holes to control the proximal clamping piece 20.

In other embodiments, the connecting structure 26 may be at least one connecting ring that is protrusively disposed on a surface of the proximal clamping piece 20 close to the fixing base 10, and each control part 230 threads the connecting ring to control the proximal clamping piece 20. The connecting ring and the proximal clamping piece 20 may be of an integrated or non-integrated structure. Specifically, in some embodiments, the connecting ring is integrally molded with the proximal clamping piece 20 by machining, stamping, or the like. In some other embodiments, the connecting ring may be a part with a connecting hole and be combined with the proximal clamping piece 20 through crimping, bonding, welding, or the like.

When each proximal clamping piece 20 is provided with at least two connecting structures 26, preferably, a line of centers of the at least two connecting structures 26 is located at or close to an axial center line of the proximal clamping piece 20 (i.e., a center line of the proximal clamping piece 20 in a direction from the connecting end 21 to the free end 22), thereby preventing the pulling force, applied by the control part 230, on the corresponding proximal clamping piece 20 from driving the proximal clamping piece 20 to distort around its axial center line.

Furthermore, a second surface of each proximal clamping piece 20 facing the corresponding distal clamping piece 30 is provided with a clamping reinforcer to increase a friction force between the proximal clamping piece 20 and the valve, thereby increasing a clamping force of the valve clip 100 to the valve. Specifically, in this embodiment, the clamping reinforcer refers to two rows of barbs 27 disposed at two opposite sides of the second surface. The barbs 27 may be integrally formed on the proximal clamping piece 20, or may be made of a material the same as or different from that of the proximal clamping piece 20 and then connected to the second surface of the proximal clamping piece 20. An included angle between an extension direction of each barb 27 and the second surface is less than or equal to 90 degrees, preferably 30-60 degrees, so that a clamping force of the valve clip 100 to the valve is enhanced.

In other embodiments, the clamping reinforcer may be structures such as ribs, bosses, or other irregularly distributed protrusions arranged on the second surface protrusively, or may be a rough surface at least partially covering the second surface, or a combination thereof, for improving the clamping force to the valve.

Preferably, each proximal clamping piece 20 may be applied with an active drug or define at least an opening 28.

It is to be noted that, the control part 230 is tensioned to the proximal end, such that the free end 22 of the proximal clamping piece 20 may be controlled to be closed relative to the fixing base 10, and the free end 22 of the proximal clamping piece 20 may fit the surface of the fixing base 10 to facilitate delivery. After the free end 22 is released from control of the control part 230, the free end 22 of the proximal clamping piece 20 is released, the proximal clamping piece 20 recovers due to its elastic memory property and restores to the natural state, and the free end 22 of the proximal clamping piece 20 is opened relative to the fixing base 10, to press the leaflets to the corresponding distal clamping piece 30. Preferably, the included angle between the two proximal clamping pieces 20 in a natural opening state should be slightly greater than that between the two distal clamping pieces 30 so that a stable clamping force can be applied. That is, an included between each proximal clamping piece 20 and the fixing base 10 is greater than or equal to an included angle between the distal clamping piece 30, on the corresponding side and opening to the utmost extent, and the fixing base 10, so that a certain clamping force is ensured between the proximal clamping piece 20 and the distal clamping piece 30 to clamp the leaflets between the two. In this embodiment, an included angle between a length direction of the proximal clamping piece 20 and the axial direction of the fixing base 10 is in a range of 0 degree to 150 degrees. That is, in a natural state, the included angle between the two proximal clamping pieces 20 may be up to 300 degrees, and a range of an opening angle between the two proximal clamping pieces 20 is preferably 0-240 degrees, and more preferably 160-200 degrees.

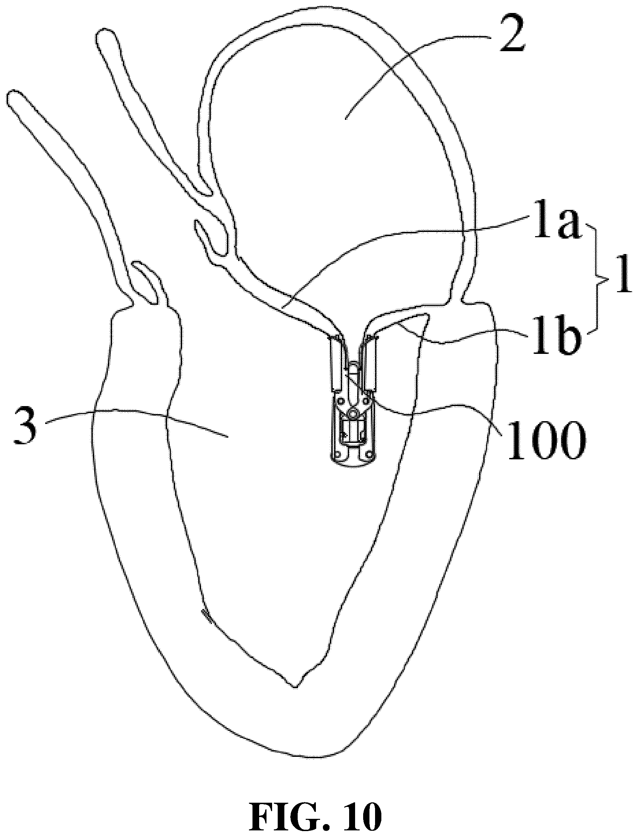

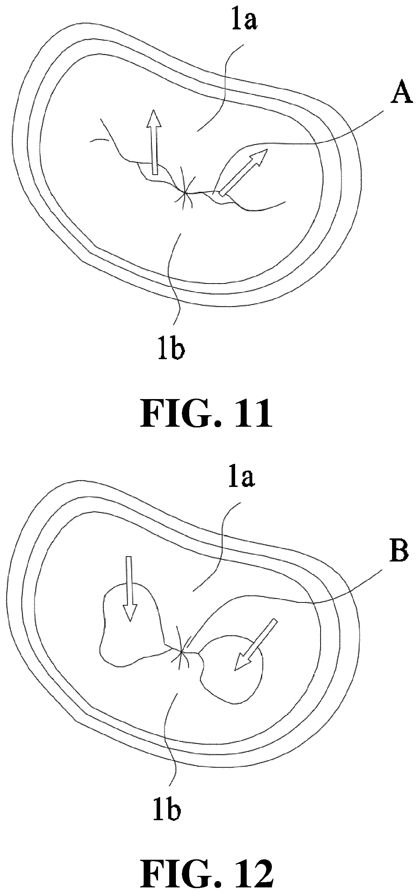

As mentioned above, the valve clip 100 provided in this embodiment can be used to reduce or treat "mitral valve regurgitation". Specifically, referring to FIG. 10 to FIG. 12 together, the valve clip 100 is placed at the position where the anterior leaflet 1a and the posterior leaflet 1b of the mitral valve cannot achieve coaptation properly. A corresponding pair of the proximal clamping piece 20 and the distal clamping piece 30 clamps the edge of the anterior leaflet 1a of the mitral valve, and the other pair of the proximal clamping piece 20 and the distal clamping piece 30 clamps the edge of the posterior leaflet 1b of the mitral valve. In this way, the anterior leaflet 1a and the posterior leaflet 1b of the mitral valve are clamped together at the positions where they cannot achieve coaptation properly. Arrow directions shown in FIG. 11 and FIG. 12 are directions of blood flow. As shown in FIG. 11, during systole, the anterior leaflet 1a and the posterior leaflet 1b are contracted, the opening area A of the mitral valve is reduced or the mitral valve can be completely closed, thereby reducing or treating the "mitral valve regurgitation". As shown in FIG. 12, during diastole, the anterior leaflet 1a and the posterior leaflet 1b achieve coaptation only at a position B where the valve clip 100 is clamped, and the other positions of the anterior leaflet 1a and the posterior leaflet 1b are still in normal diastole, which allows blood to enter the left ventricle from the left atrium, thereby ensuring normal circulation of blood.

It can be understood that, because the two leaflets of the mitral valve are always in an opening and closing movement state, it is relatively difficult to clamp, and only one leaflet can be clamped successfully, and the other leaflet may be clamped partially, which may result in that the two leaflets of the mitral valve are in coaptation at a non-ideal position or the leaflet that is partially clamped finally slip off from the valve clip 100. At this point, repetition of operation has to be required. That is, the control part 230 is tensioned to the proximal end to control the proximal clamping piece 20 that presses the leaflet which is partially clamped to be closed relative to the fixing base 10, the position of the valve clip 100 is adjusted to make the distal clamping piece 30 located at a proper position of the leaflets, and then the proximal clamping piece 20 is released from control of the control part 230, the proximal clamping piece 20 is opened relative to the fixing base 10 and then moves closer to the corresponding distal clamping piece 30 to re-clamp the leaflets, until the leaflets of the mitral valve are firmly clamped, so that regurgitation disappears or achieves a slightest state.

Referring to FIG. 5 and FIG. 6, in order to prevent the pulling force, applied by the control part 230, on the corresponding proximal clamping piece 20 from causing bending of the delivery sheath 210 and swinging of the valve clip 100, in this application, the control part 230 threads the at least one pair of first cavities 211 to extend out of the body of the patient, where the at least one pair of first cavities 211 of the delivery sheath 210 are disposed symmetrically about the axis of the delivery sheath 210. When the control part 230 controls the corresponding proximal clamping piece 20 to be closed relative to the fixing base 10, the resultant F of the pulling forces, applied by the control parts 230, on the corresponding proximal clamping pieces 20 is exerted on the axis of the delivery sheath 210. As such, the resultant F generates a torque of zero to the valve clip 100, thereby avoiding bending of the delivery sheath 210 and swinging of the valve clip 100, and the proximal clamping pieces 20 can be quickly and accurately released by the operator to re-clamp the valve, without frequently adjusting the position of the valve clip 100, thereby facilitating reduction of operation time and improvement of operation efficiency, and preventing the valve clip 100 from swinging to hook the chordae tendineae or the tissue to hurt the patient.

As shown in FIG. 5, in this embodiment, the delivery sheath 210 defines two pairs of first cavities 211. Each pair of the first cavities 211 is threaded with the control part 230 that is used to control the corresponding proximal clamping piece 20. That is, in this embodiment, two control parts 230 that are respectively used to control two proximal clamping pieces 20 thread two different pairs of first cavities 211.

Each control part 230 is a single control wire. The control wire threads the corresponding proximal clamping piece 20, both ends of the control wire respectively thread one pair of first cavities 211 and extend out of the body of the patient. Specifically, one first cavity 211 of the one pair of first cavities 211 acts as an incoming passage for the control wire, and the other first cavity 211 of the one pair of first cavities 211 acts as an outgoing passage for the control wire. That is, the incoming passage and the outgoing passage of the control wire are symmetrical about the axis of the delivery sheath 210. As shown in FIG. 5, in this embodiment, the control wire reaches the corresponding proximal clamping piece 20 along the incoming passage, passes through the connecting structure 26 on the proximal clamping piece 20, and then is reversely bent and then extends out of the body of the patient through the outgoing passage. As shown in FIG. 6, when one control wire is pulled by the operator toward the proximal end to control one corresponding proximal clamping piece 20 to be closed relative to the fixing base 10, pulling forces, applied by the control wire that is bent into two parts, on the corresponding proximal clamping piece 20 are respectively F1 and F2. F1 and F2 are symmetrical about the axis of the delivery sheath 210. A resultant F of the pulling forces is exerted on the axis of the delivery sheath 210. As such, the resultant F generates a torque of zero to the valve clip 100, thereby avoiding bending of the delivery sheath 210 and swinging of the valve clip 100, and the position of the valve clip 100 can be rapidly adjusted and the proximal clamping pieces 20 can be accurately released by the operator to re-clamp the valve, thereby facilitating reduction of operation time and improvement of operation efficiency, and preventing the valve clip 100 from swinging to hurt the patient.

The control wire is a single wire or multiple wires twisted. The wire is selected from a nickel-titanium wire, a stainless steel wire, or a high-strength polymer wire. In this embodiment, the control wire is a single nickel-titanium wire, that is, the control part 230 is a single nickel-titanium wire.

The first cavity 211 is in a shape of circular, square, polygonal, or other irregular shapes. In this embodiment, the first cavity 211 is a circular cavity.

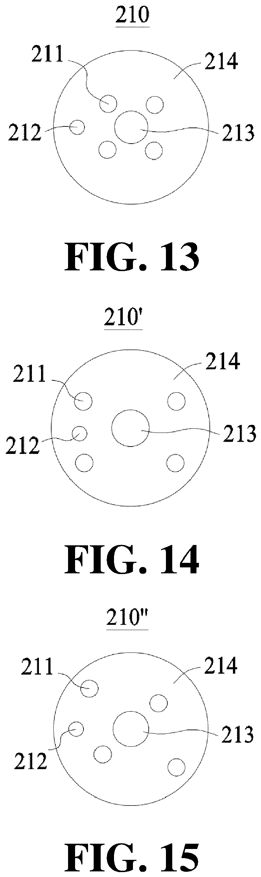

Referring to FIG. 13, in this embodiment, distances from the axes of two pairs of first cavities 211 to the axis of the delivery sheath 210 are the same. Distances from each first cavity 211 to two first cavities 211 of the other pair of first cavities 211 are the same. In other words, the two pair of first cavities 211 are distributed in square. Apparently, in other embodiments, distances from each first cavity 211 to two first cavities 211 of the other pair of first cavities 211 may be different, that is, the two pairs of first cavities 211 are distributed in rectangle, as shown in FIG. 14.

In other embodiments, distances from the axes of two pairs of first cavities 211 to the axis of the delivery sheath 210 may be different, that is, the two pairs of first cavities 211 are distributed in parallelogram, as shown in FIG. 15.

In other embodiments, the delivery sheath 210 may define only a pair of first cavities 211 that are respectively used to control two control wires of the two proximal clamping pieces 20 to thread the pair of first cavities 211. Specifically, the same first cavity 211 in the pair of first cavities 211 is used as an incoming passage of the two control wires, and the other first cavity 211 in the pair of first cavities 211 is used as an outgoing passage of the two control wires.

Furthermore, as shown in FIG. 5 and FIG. 13, in this embodiment, the valve clamping system further includes a pressure sensor (not shown in the figure), and the delivery sheath 210 defines a second cavity 212 extending axially. The pressure sensor movably penetrates the second cavity 212 and can extend out of the second cavity 212 from a distal end of the second cavity 212. When the delivery device 200 delivers the valve clip 100 to the left ventricle of the heart and the two leaflets of the mitral valve are clamped by the valve clip 100, the pressure sensor may be inserted to the left atrium of the heart from the distal end of the second cavity 212, so that internal pressure value of the heart can be fed back in real time to confirm alleviation of the "mitral valve regurgitation" of the patient, so as to determine whether it is necessary to adjust the position of the valve clip 100 and re-clamp the leaflets of the mitral valve to optimize the treatment effect.

It can be understood that the pressure sensor may be assembled in the second cavity 212 of the delivery sheath 210 when leaving the factory, or other compatible commercial pressure sensors may be used, which are assembled in the second cavity 212 of the delivery sheath 210 by the operator before an operation.

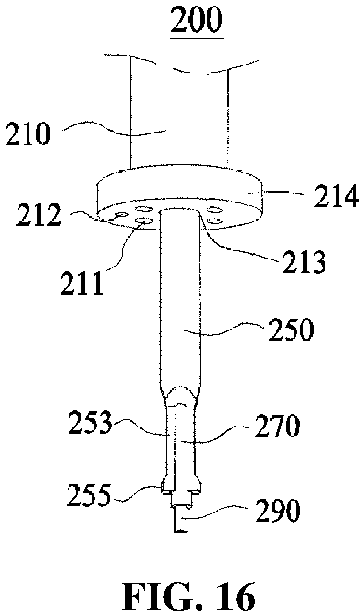

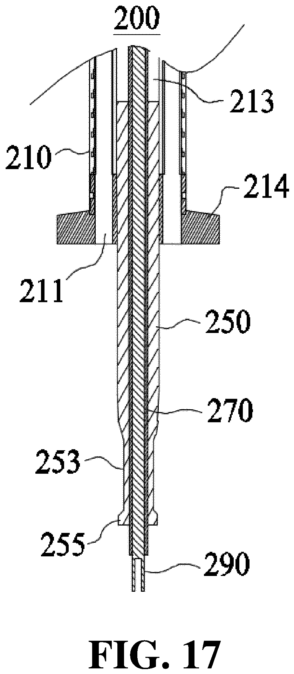

Referring to FIG. 5, FIG. 7, FIG. 16, and FIG. 17, the valve clamping system provided in this embodiment includes the valve clip 100 and the delivery device 200. The valve clip 100 is delivered to the mitral valve through the delivery device 200 and is adjusted to a proper position of the mitral valve. The delivery device 200 includes an operating handle and a delivery component. A proximal end of the delivery component is connected to the operating handle, and a distal end of the delivery component is detachably connected to the valve clip 100.

The delivery component includes the foregoing delivery sheath 210 and the control parts 230 threading the first cavities 211 of the delivery sheath 210. Furthermore, the delivery component also includes a connector 250 inserted at the distal end of the delivery sheath 210, a bushing 270 movably and coaxially inserted in a tubular cavity of the connector 250, and a mandrel 290 movably and coaxially inserted in the bushing 270. By the operating handle disposed outside the body of the patient, the control parts 230 can be tensioned and the bushing 270 and the mandrel 290 can be respectively driven to move or rotate relatively, by the operator.

The delivery sheath 210 defines a third cavity 213 in the axial direction. The connector 250 is inserted at a distal end of the third cavity 213. The bushing 270 and the mandrel 290 pass through the third cavity 213 to extend out of the body of the patient. Preferably, in the embodiment, the delivery sheath 210 is provided with a fastener 214 at the distal end. The connector 250, the bushing 270, and the mandrel 290 that are coaxially nested together are inserted in the third cavity 213 of the delivery sheath 210 by using the fastener 214, and thus connection strength is improved. It can be understood that the fastener 214 defines multiple through holes that respectively communicate with the first cavities 211, the second cavity 212, and the third cavity 213.

The delivery sheath 210 may be a multi-cavity tube made of a biocompatible metal material or a polymer material through integral molding, or may be a multi-cavity tube formed of multiple single tubes that are made of a biocompatible metal material or a polymer material through crimping, welding or bonding. The metal material is selected from stainless steel, aluminum alloy, or cobalt-chromium alloy, and the polymer material is selected from polycarbonate, polyethylene, or polyamide. In this embodiment, a pushing catheter 210 is a multi-cavity tube formed by integral molding and defines multiple cavities extending in the axial direction of the pushing catheter 210. The multiple cavities form the at least one pair of first cavities 211, the second cavity 212, and the third cavity 213 described above.

The connector 250 is in a substantially tubular shape. The connector 250 is provided with two connecting rods 253 axi-symmetrically disposed at the distal end. Each connecting rod 253 is provided with a snap-in joint 255 at a distal end for detachable connection with the fixing base 10. Inner surfaces opposite mutually of the two axi-symmetrical connecting rods 253 are cambered surfaces in smooth transition and connection with a cylindrical surface in the tubular cavity of the connector 250. A distance from the cambered surface of each connecting rod 253 to the axis of the connector 250 is gradually reduced from the proximal end to the distal end. In other words, the distal end of each connecting rod 253 inclines to the axis of the connector 250, and the two connecting rods 253 are gradually closed from the proximal end to the distal end.

The connecting rods 253 are made of an elastic material. When the connecting rods 253 are exerted with an outward pushing force in a radial direction of the connector 250, the distal ends of the connecting rods 253 are expanded outwards. The bushing 270 of the delivery device 200 movably penetrates the tubular cavity of the connector 250. The bushing 270 is driven to move to the distal lend and then pushes the connecting rods 253 outwards in the radial direction. The snap-in joints 255 of the connecting rods 253 are expanded outwards and engaged in the locking holes 111 of the fixing base 10, so that the delivery device 200 is connected to the fixing base 10 through the connector 250. It can be understood that the bushing 270 is retracted to the proximal end, the connecting rods 253 recover due to their own elasticity, the distal ends of the connecting rods 253 are closed to the axis of the connector 250, the snap-in joints 255 are released from the corresponding locking holes 111, so that the delivery device 200 is disconnected from the fixing base 10.

The mandrel 290 and the push rod 40 are in detachable connection, so that the push rod 40 is driven to axially slide along the fixing base 10 to drive the distal clamping pieces 30 to be opened or closed relative to the fixing base 10. In this embodiment, the mandrel 290 is a round rod body with an internal threaded hole (not shown in the figure) at the distal end. The internal threaded hole is used for threaded connection with an external thread at the proximal end of the push rod 40.

In other embodiments, the delivery component may not include the bushing 270. An overall diameter of the mandrel 290 or a diameter of the distal part of the mandrel 290 is relatively large, then the distal part of the mandrel 290 can directly push the snap-in joints 255 at the distal end of the connector 250 to expand outward. That is, the mandrel 290 may be used to push the snap-in joints 255 at the distal end of the connector 250 to expand outwards and may also be used to drive the push rod 40 to axially slide along the fixing base 10.

It is to be noted that the delivery component and the valve clip 100 may be delivered into the body of the patient by means of a related bendable sheath.

The following takes a mitral valve repair process as an example to illustrate an operation method of the valve clamping system of this application, which mainly includes the following steps.

At the first step, the delivery component is detachably connected to the valve clip 100. Specifically, the bushing 270 is used to push the snap-in joints 255 at the distal end of the connector 250 outwards, so that the snap-in joints 255 are engaged in the locking holes 111 of the fixing base 10, and the fixing base 10 and the connector 250 are kept in a connected state. The mandrel 290 is rotated to be screwed to the push rod 40. The control parts 230 are tensioned to the proximal end to control the proximal clamping pieces 20 to be closed relative to the fixing base 10, so that the free ends 22 of the proximal clamping pieces 20 fit the surface of the fixing base 10. Then, by means of the operating handle, the mandrel 290 is moved to the distal end to drive the push rod 40 to axially slide to the distal end, to drive the distal clamping pieces 30 to be closed relative to the fixing base 10, so that the valve clip 100 is in a fully closing state, and the proximal clamping pieces 20 and the distal clamping pieces 30 both fit the surface of the fixing base 10 and are kept in a closing state.

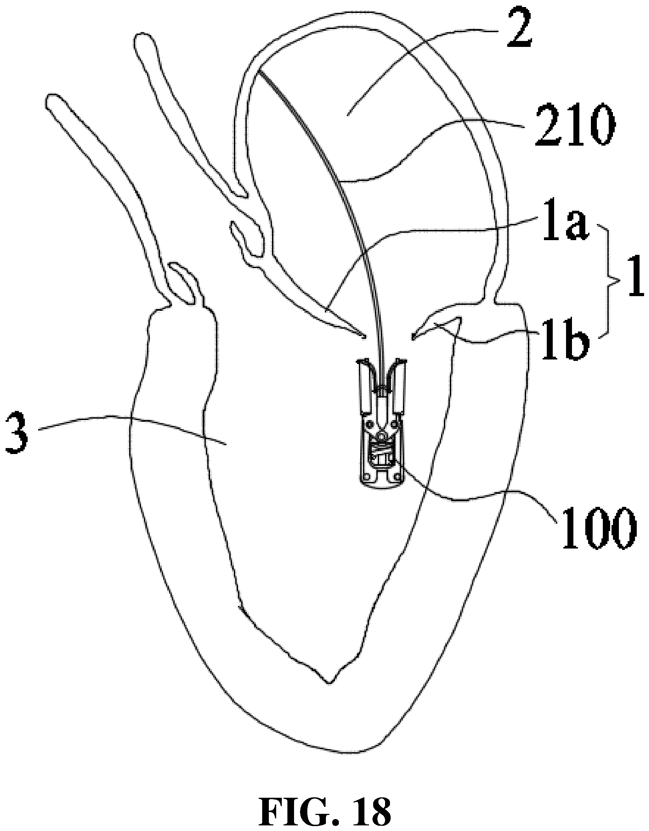

At the second step, by a transseptal pathway, the delivery component and the valve clip 100 are pushed via the bendable sheath from the left atrium to enter the left ventricle through the mitral valve, as shown in FIG. 18.

At the third step, the relative position of the valve clip 100 and the mitral valve is adjusted so that the valve clip 100 approaches the anterior leaflet 1a and posterior leaflet 1b of the mitral valve.

At the fourth step, through the operating handle, the mandrel 290 is moved to the proximal end to drive the push rod 40 to slide to the proximal end, so as to drive the distal clamping pieces 30 to be opened relative to the fixing base 10, and a direction of the valve clip 100 is adjusted so that the distal clamping pieces 30 are perpendicular to a coaptation line of the mitral valve.

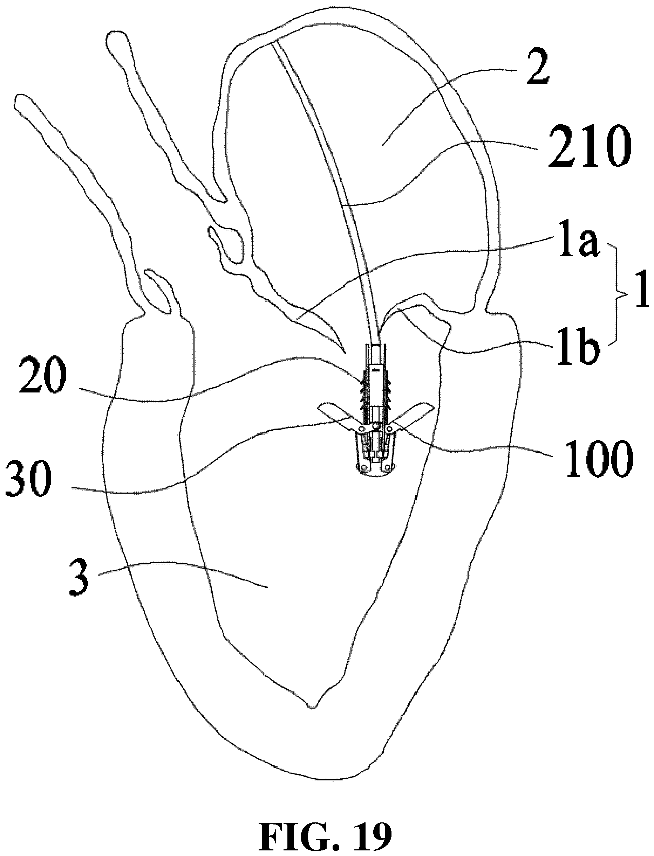

At the fifth step, the entire valve clip 100 is retracted to the proximal end, and the distal clamping pieces 30 support the leaflets at one side of the left ventricle, as shown in FIG. 19.

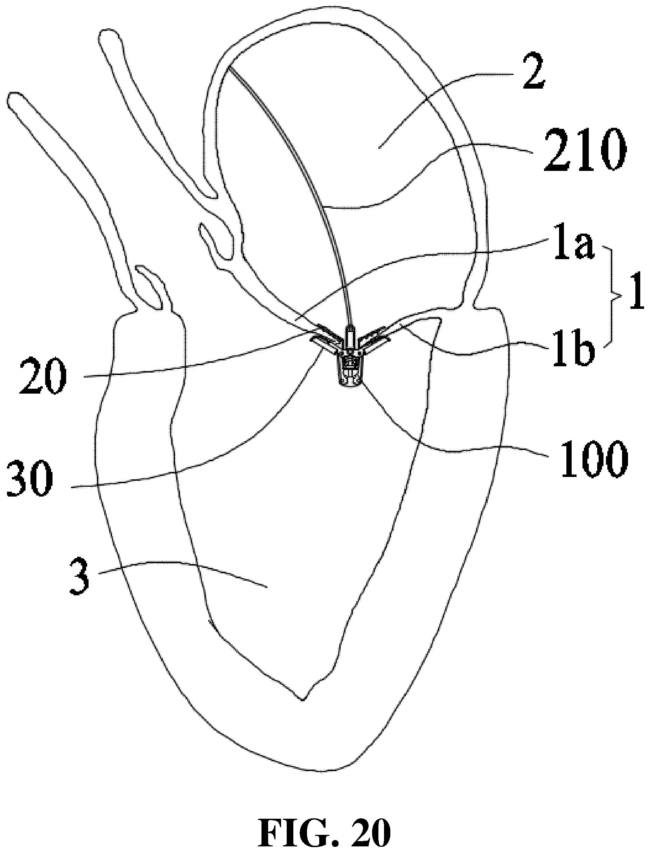

At the sixth step, the corresponding proximal clamping piece 20 is released from control of each control part 230. The proximal clamping pieces 20 on two sides are released sequentially, and the proximal clamping piece 20 on each side presses the leaflets at the side of the atrium and matches with the distal clamping piece 30 on the side to clamp the leaflets. In a process of releasing the proximal clamping piece 20 on the single side, an incoming end and an outgoing end of the control part 230 corresponding to the proximal clamping piece 20 that needs to be tensioned outside the body are simultaneously pulled by the operator, and pulling forces that act on the incoming wire and the outgoing wire are equivalent. The incoming passage and the outgoing passage are located in the two first cavities 211 that are symmetrical about the axis of the delivery sheath 210, so that a resultant of the pulling forces, applied by the control part 230, on the corresponding proximal clamping piece 20 is exerted on axis of the delivery sheath 210, to avoid bending of the delivery sheath 210 and swinging of the valve clip 100. As such, each proximal clamping piece 20 can be independently controlled to expand relative to the fixing base 10, the anterior leaflet 1a and the posterior leaflet 1b of the mitral valve are respectively clamped between the corresponding proximal clamping piece 20 and the distal clamping piece 30, thereby achieving complete clamping of the leaflets, as shown in FIG. 20.

At the seventh step, the pressure sensor extends out of the distal end of the second cavity 212 of the delivery sheath 210 to enter the left atrium, the pressure value inside the heart is detected to confirm alleviation of the "mitral valve regurgitation" of the patient, so as to determine whether it is necessary to adjust the position of the valve clip 100 and re-clamp the leaflets to optimize the treatment effect. For the case where the leaflets need to be clamped again, the control part 230 is tensioned toward the proximal end to control the proximal clamping piece 20 pressed on the leaflets whose clamping position is not ideal to be closed relative to the fixing base 10. The position of the valve clip 100 is adjusted to make the distal clamping piece 30 located at an appropriate position of the leaflets, and then the proximal clamping piece 20 is released from control of the control part 230. As such, the proximal clamping piece 20 is expanded relative to the fixing base 10 and then moves closer to the distal clamping piece 30 to re-clamp the leaflets until the mitral valve leaflets are completely clamped.

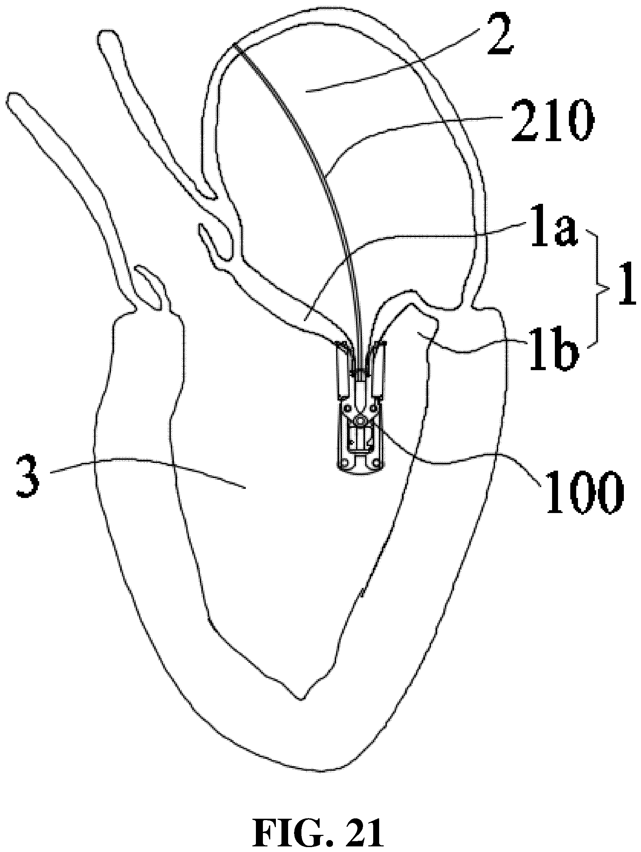

At the eighth step, the mandrel 290 is moved to the distal end again to drive the push rod 40 to axially move to the distal end, thereby driving the distal clamping pieces 30 to be closed relative to the fixing base 10 until the valve clip 100 is fully closed, as shown in FIG. 21.

At the ninth step, the mandrel 290 is controlled to rotate by the operating handle to unlock a threaded connection between the mandrel 290 and the push rod 40, and then the bushing 270 and the mandrel 290 are retracted toward the proximal end until the snap-in joints 255 at the distal end of the connector 250 are unlocked and separated from the locking holes 111 of the fixing base 10, and the valve clip 100 is completely separated from the delivery component. Finally, the delivery component is withdrawn from the body of the patient. Here, the valve clip 100 pulls the anterior leaflet 1a and the posterior leaflet 1b of the mitral valve toward each other to obtain a double-orifice mitral valve and complete the edge-to-edge repair of the mitral valve, and the valve clip 100 is indwelled in the body of the patient, as shown in FIG. 10.

It can be understood that the valve clamping system of this application can also use a pathway such as a transapical pathway to deliver the valve clip to the mitral valve.

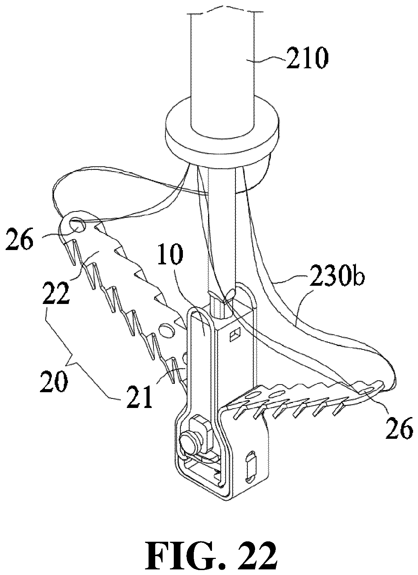

Referring to FIG. 22, the structure of the valve clamping system provided in a second embodiment of this application is similar to that of the valve clamping system in the first embodiment. The difference lies in that: in the second embodiment, each control part 230b includes at least two control wires, that is, each proximal clamping piece 20 is controlled by at least two control wires to realize closing or opening relative to the fixing base 10. Specifically, in this embodiment, each control part 230b includes two control wires, and the two control wires thread the same pair of first cavities 211 of the delivery sheath 210. One of the first cavities 211 of the pair of first cavities 211 is used as an incoming passage of the two control wires, and the other first cavity 211 of the pair of first cavities 211 is used as an outgoing passage of the two control wires. The incoming passage and the outgoing passage of each control wire are still symmetrical about the axis of the delivery sheath 210, so as to prevent the valve clip 100 from swinging.

It can be understood that each control part 230b includes at least two control wires, which can improve a control force of each control part 230b on the corresponding proximal clamping piece 20. That is, at least two control wires can provide a greater pulling force, making the proximal clamping piece 20 closer to the fixing base 10 to the utmost extent, and preventing the proximal clamping piece 20 from being released in advance due to insufficient pulling force.

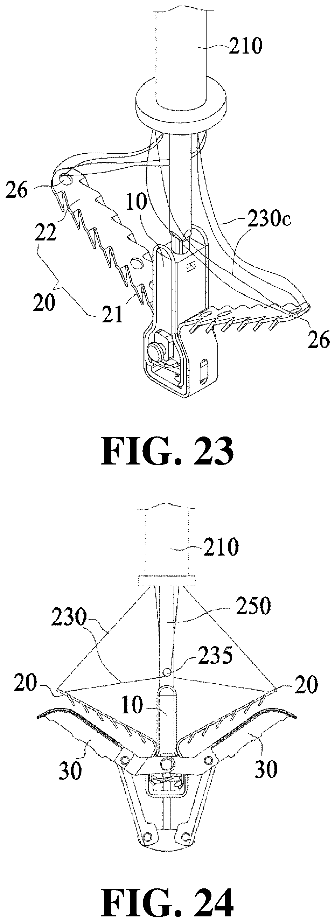

Referring to FIG. 23, the structure of the valve clamping system provided in a third embodiment of this application is similar to that of the valve clamping system in the first embodiment. The difference lies in that: in the third embodiment, each control part 230c includes at least one pair of control wires, that is, each proximal clamping piece 20 is controlled by at least one pair of control wires to realize closing and opening relative to the fixing base 10. Each pair of control wires can thread the corresponding proximal clamping piece 20 and extends through one pair of first cavities 211 of the delivery sheath 210, and is operable to extend out of the body of the patient.

In this embodiment, each control part 230c includes a pair of control wires, and the pair of control wires threads a pair of first cavities 211 of the delivery sheath 210. Specifically, in one pair of control wires used to control the proximal clamping piece 20, one first cavity 211 of the one pair of first cavities 211 acts as both an incoming passage and an outgoing passage for one control wire, the other first cavity 211 of the one pair of first cavities 211 acts as both an incoming passage and an outgoing passage for the other control wire. That is, the pair of control wires used to control each proximal clamping piece 20 is symmetrically arranged about the axis of the delivery sheath 210, so as to prevent the valve clip 100 from swinging.

In other embodiments, each control part 230c may include at least two pairs of control wires, and each pair of the at least pair of control wires threads a pair of first cavities 211 of the delivery sheath 210 and extends out of the body of the patient. Among the at least two pairs of control wires used to control each proximal clamping piece 20, the two different pairs of control wires can pass through the same pair of first cavities 211 to extend out of the body of the patient, or can respectively pass through a pair of first cavities 211 to extend out of the body of the patient.

It can be understood that each control part 230c includes at least one pair of control wires, which can improve a control force of each control part 230c on the corresponding proximal clamping piece 20. That is, at least one pair of control wires can provide a greater pulling force, making the proximal clamping piece 20 closer to the fixing base 10 to the utmost extent, and preventing the proximal clamping piece 20 from being released in advance due to insufficient pulling force.

Referring to FIG. 24, the structure of the valve clamping system provided in a fourth embodiment of this application is similar to that of the valve clamping system in the first embodiment. The difference lies in that: in the fourth embodiment, at least one winding post 235 is disposed on the connector 250 or the fixing base 10. The incoming end and/or the outgoing end of the control wire (that is, the control part 230) passes over the at least one winding post 235 to be operable to extend out of the body of the patient. Specifically, in this embodiment, two winding posts 235 are protrusively disposed on an outer wall of the connector 250 and are symmetrical about the axis of the connector 250. After the incoming end of the control wire threads the corresponding proximal clamping piece 20, the outgoing end of the control wire passes over one winding post 235 and then extends upward into the outgoing passage of the delivery sheath 210 until the outgoing end of the control wire extends out of the body of the patient. In this embodiment, the two control wires used to control the two proximal clamping pieces 20 respectively pass over different winding posts 235.

In other embodiments, the incoming end of each control wire can pass over the winding post 235 and then threads the corresponding proximal clamping piece 20, and the outgoing end of the control wire directly extends upward into the outgoing passage of the delivery sheath 210 until the outgoing end of the control wire extends out of the body of the patient.

In other embodiments, the incoming end and the outgoing end of each control wire can pass over the winding post 235 and respectively extend out of the body of the patient through the incoming passage and the outgoing passage of the delivery sheath 210.

In other embodiments, only one winding post 235 may be provided for controlling the incoming and/or outgoing ends of the two control wires of different proximal clamping pieces 20 to wind around the same winding post 235.

Preferably, at least one winding groove may be defined on the winding post 235, and the control wire is partially wound in the at least one winding groove. When the incoming end and outgoing end of the control wire or the incoming end and/or outgoing end of the two control wires used to control different proximal clamping pieces 20 are simultaneously wound on the winding post 235, the winding post 235 defines multiple winding grooves at intervals, to avoid mutual influence of the control wires.

It can be understood that the incoming end and/or the outgoing end of the control wire are wound on the winding post 235. When the control wire is tensioned toward the proximal end to control the corresponding proximal clamping piece 20 to be closed relative to the fixing base 10, the incoming end and/or the outgoing end of the control wire slide around the winding post 235, the winding post 235 acts as a fixed pulley, which helps the operator to use a smaller pulling force to tension the proximal clamping piece 20, thereby preventing the proximal clamping piece 20 from being released early.

It is to be noted that the above content is described with the valve clip being used for alleviating or treating "mitral valve regurgitation" as an example. It can be understood that in other embodiments, the valve clip can also be used to alleviate or treat "tricuspid valve regurgitation", and the principle and structure thereof are substantially the same as those of the valve clip used to resolve the problem of "mitral valve regurgitation" in embodiments of this application. That is, multiple clamps are formed by multiple pairs of proximal clamping pieces and distal clamping pieces, and each clamp can clamp a leaflet respectively, which will not be elaborated here.

Apparently, in other embodiments, the valve clip provided in this application can also be applied to other minimally invasive surgical operations in which more than three flap-shaped valves are required to be clamped together.

The above description are embodiments of this application, and it is noted that various improvements and modifications can be made without departing from the principle of the application to those of ordinary skill in the art, and the improvement and the modification are also considered as the protection scope of the this application.

## Claims

1. A valve clamping system capable of being independently controlled, comprising:
a valve clip comprising a fixing base and proximal clamping pieces capable of being folded or unfolded relative to the fixing base; and
a delivery device configured to deliver the valve clip, wherein the delivery device comprises a delivery sheath and control parts, each control part is connected to one corresponding proximal clamping piece, so as to control the proximal clamping pieces to be folded or unfolded relative to the fixing base;
the delivery sheath defines at least one pair of first cavities that are symmetrical about an axis of the delivery sheath and axially extend, and each control part movably threads the at least one pair of first cavities.

2. The valve clamping system of claim 1, wherein each control part comprises at least one control wire, wherein the at least one control wire threads the corresponding proximal clamping piece, and both ends of the at least one control wire respectively thread one pair of first cavities and extend out of a body of a patient.

3. The valve clamping system of claim 2, wherein for each of the at least one control wire, one first cavity of the one pair of first cavities acts as an incoming passage for the control wire, and the other first cavity of the one pair of first cavities acts as an outgoing passage for the control wire.

4. The valve clamping system of claim 1, wherein each control part comprise at least one pair of control wires, each pair of the at least one pair of control wires threads the corresponding proximal clamping piece and extends through one pair of first cavities of the delivery sheath, and is operable to extend out of a body of a patient.

5. The valve clamping system of claim 4, wherein for each pair of the at least one pair of control wires, one first cavity of the pair of first cavities acts as both an incoming passage and an outgoing passage for one control wire, and the other first cavity of the pair of first cavities acts as both an incoming passage and an outgoing passage for the other control wire.

6. The valve clamping system of claim 2 or 4, wherein
a distal end of the delivery device is provided with a connector, the delivery device is detachably connected with the fixing base via the connector;
the connector or the fixing base is provided with at least one winding post; and
an incoming end and/or an outgoing end of the control wire passes over the at least one winding post to be operable to extend out of a body of a patient.

7. The valve clamping system of claim 6, wherein the control wire is a single wire or multiple wires twisted, the wire is selected from a nickel-titanium wire, a stainless steel wire, or a high-strength polymer wire.

8. The valve clamping system of claim 1, wherein the delivery sheath defines two pairs of first cavities, distances from axes of two pairs of first cavities to the axis of the delivery sheath are the same or different, and each pair of first cavities is threaded with one control wire.

9. The valve clamping system of claim 1, wherein each proximal clamping piece is provided with at least one connecting structure that is used to be connected to the control part.

10. The valve clamping system of claim 9, wherein the connecting structure is disposed at an end of the proximal clamping piece which is away from the fixing base when the proximal clamping piece is unfolded relative to the fixing base.

11. The valve clamping system of claim 9, wherein each proximal clamping piece is provided with at least two connecting structures, a line of centers of the at least two connecting structures is located at or close to an axial center line of the proximal clamping piece.

12. The valve clamping system of any of claims 9 to 11, wherein
the connecting structure is a connecting hole defined in the proximal clamping piece; or
the connecting structure is a connecting ring protrusively disposed on the proximal clamping piece.

13. The valve clamping system of claim 1, wherein
the delivery sheath is a multi-cavity tube made of a biocompatible metal material or a polymer material through integral molding; or
the delivery sheath is a multi-cavity tube formed of multiple single tubes that are made of a biocompatible metal material or a polymer material through crimping, welding or bonding.

14. The valve clamping system of claim 1, wherein the valve clip further comprises distal clamping pieces capable of being folded or unfolded relative to the fixing base, a leaflet accommodating space is defined between the proximal clamping pieces and the distal clamping pieces.

15. The valve clamping system of claim 14, wherein the proximal clamping pieces are made of a material having a shape memory function, and a hardness of the distal clamping pieces is greater than that of the proximal clamping pieces.

16. The valve clamping system of claim 1, wherein the valve clamping system further comprises a pressure sensor, the delivery sheath defines a second cavity extending axially, and the pressure sensor penetrates the second cavity and is operable to extend out of a delivery device from a distal end of the second cavity.
